Europäisches Patentamt

European Patent Office

Office européen des brevets

(19)

(11) **EP 0 695 801 A2**

(12) **EUROPEAN PATENT APPLICATION**

(43) Date of publication:
07.02.1996 Bulletin 1996/06

(51) Int. Cl.⁶: $C12N\ 1/06$, $A61K\ 7/06$, $A61K\ 7/48$

(21) Application number: 95112300.9

(22) Date of filing: 04.08.1995

(84) Designated Contracting States:
DE FR GB

(30) Priority: 05.08.1994 JP 204253/94

(71) Applicants:
• SEIWA KASEI CO., LTD.
Higashiosaka-shi Osaka-fu (JP)
• KIRIN BEER KABUSHIKI KAISHA
Tokyo-To (JP)

(72) Inventors:
• Yoshioka, Masato,
c/o Seiwa Kasei Co., Ltd.
Higashi-Osaka-shi, Osaka-fu (JP)
• Adachi, Takashi,
c/o Seiwa Kasei Co., Ltd.
Higashi-Osaka-shi, Osaka-fu (JP)
• Matsumoto, Hajime,
c/o Kırin Beer K.K.
Tokyo-to (JP)

(74) Representative: Hansen, Bernd, Dr. Dipl.-Chem. et
al
D-81925 München (DE)

(54) **Yeast protein derived peptide compositions, a process for preparing them and their use**

(57)    A novel protein hydrolysate having properties superior to those of the conventional protein hydrolysates are provided.

Yeast cells are subjected to the heating treatment and the treatment with a cell wall lytic enzyme, followed by separation and purification to give a yeast protein, which is then hydrolyzed to produce a yeast protein derived peptide composition.

The yeast protein derived peptide composition, having an average molecular weight from 200 to 5,000, is particularly suitable for a component of a cosmetic.

The yeast protein derived peptide composition is easy to control the molecular weight on the preparation of a peptide. A yeast protein derived peptide composition having a stable molecular weight can be obtained with a good reproducibility, and the yeast protein derived peptide composition thus obtained is light in color, low in smell and excellent in stability during storage, gives hair and skin gloss and moistness, and protect hair and skin.

FIG. I

EP 0 695 801 A2

**Description**

BACKGROUND OF THE INVENTION

Field of the Invention

The present invention relates to a yeast protein derived peptide composition, a process for preparing it, and its use.

Background Art

Protein hydrolysates protect hair and skin, and exhibit a moisturizing effect to afford gloss and moistness to hair and skin, so that these materials have been hitherto incorporated into hair cosmetics and skin care cosmetics. As the protein sources thereof, there have been used particularly those derived from animals such as collagen, keratin or silk, or those derived from plants such as wheat, barley or corn. As the hydrolysates of proteins derived from microorganisms, it has been tried to use a yeast protein derived peptide composition as a stabilizer of emulsion for cosmetics (Japanese Patent Laid-Open Publication No. 42483/1977).

The yeast protein derived peptide composition described above has drawbacks that it is difficult to control the molecular weights on degrading the composition into peptides, and that the hydrolysate is dark brown, has a strong smell, and produces polymeric deposits during its storage, so that it has many difficulties for its application to cosmetics and thus has not been turned to practical use.

SUMMARY OF THE INVENTION

The object of the present invention is to provide a novel protein hydrolysate which has more excellent properties than those of the conventional protein hydrolysates.

BRIEF DESCRIPTION OF THE DRAWINGS

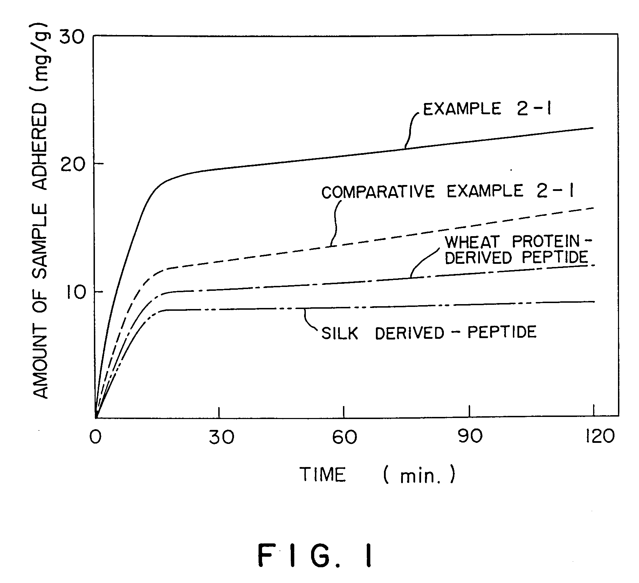

Figure 1 represents the sorption properties to hair of the yeast protein derived peptide composition of the present invention in Example 2-1, average molecular weight: 906, a yeast protein derived peptide composition, average molecular weight: 826, a wheat protein derived peptide, average molecular weight: 1,000, and a silk protein derived peptide, average molecular weight: 1,000, in Comparative Example 2-1.

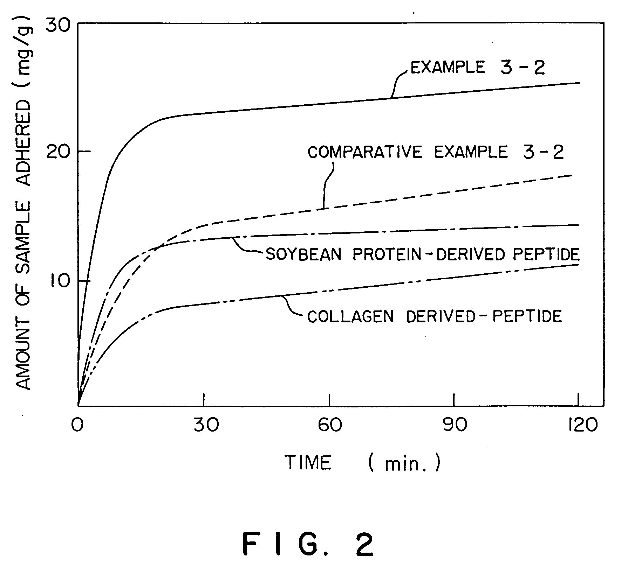

Figure 2 represents the sorption properties to hair of the yeast protein derived peptide composition of the present invention in Example 3-2, average molecular weight: 593, a yeast protein derived peptide composition, average molecular weight: 400, a collagen protein derived peptide, average molecular weight: 450, and a soybean protein derived peptide, average molecular weight: 480, in Comparative Example 3-2.

DETAILED DESCRIPTION OF THE INVENTION

The present inventors have examined a variety of processes for preparing a protein derived peptide composition which is light in color, low in smell and excellent in stability during storage. As a result, they have found that a yeast protein derived peptide composition obtained by the hydrolysis of a yeast protein which had been obtained by subjecting a yeast cell to heating and treatment with a cell wall lytic enzyme is light in color, low in smell and excellent in stability during storage as compared with a yeast protein derived peptide composition prepared by the conventional method, that it is easy to control the molecular weight of the peptide on its preparation, that a yeast protein derived peptide composition having a stable molucular weight can be produced with a good reproducibility, and that the yeast protein derived peptide composition thus obtained has excellent sorption propreties to hair and skin and an excellent moisturizing effect as compared with the conventional peptides for cosmetics. The present invention has been thus accomplished.

That is to say, the yeast protein derived peptide composition according to the present invention is that prepared by hydrolyzing a yeast protein which is obtained by subjecting yeast cells to heating treatment and treatment with a cell wall lytic enzyme, and has such excellent properties as described above.

The present invention will be described in detail below from the aspects of yeasts as protein sources, cell wall lytic enzymes used for separating a yeast protein from the yeast, a yeast protein thus obtained, a yeast protein derived peptide composition obtained by the hydrolysis of the yeast protein, the properties of the yeast protein derived peptide composition, the applications of the yeast protein derived peptide composition, and the like.

[Yeast]

As a yeast for obtaining a yeast protein in the present invention, either one of the yeasts of which the cell wall can be lysed with a cell wall lytic enzyme can be used such as yeasts belonging to Saccharomyces, Endomycopsis, Torulopsis, or Candida genera. It is also possible to use a yeast which is industrially called brewer's yeast, baker's yeast or sake yeast. It is also possible to use the residue of the aforementioned yeasts which have been used for the extraction of materials other than protein, if the residue contains the protein in such a state that it can be used for the above described treatments.

[Yeast cell wall lytic enzyme]

The yeast cell wall lytic enzyme is the generic name of enzymes which attack glucan as a component of the cell wall of a yeast cell and has the activities to relax, decompose and remove the cell wall. The yeast cell wall lytic enzymes used in the present invention are not particularly limited as far as they have the aforementioned activities, and include preferably a yeast cell wall lytic enzyme which is low in protease activity such as an enzyme produced by the microorganisms belonging to Arthrobacter or Oerskovia. Specific examples of such yeast cell wall lytic enzymes include preferably a yeast cell wall lytic enzymes produced by Arthrobacter luteus, ATCC 21606 (see US Patent Nos. 3716452 and 391750.

[Yeast protein]

In order to obtain a yeast protein from a yeast, an aqueous suspension containing about 1 - 20% by weight of yeast cells is preferably subjected to heating treatment by an appropriate means. The heating treatment may be carried out after the treatment with a cell wall lytic enzyme. Heating is preferably carried out from the viewpoint of the recovery of the yeast protein in the range from 50°C to 100°C, particularly from 70°C to 100°C. When the heating treatment is carried out before the treatment with the cell wall lytic enzyme, the heating treatment is preferably carried out in the the range from 90°C to 100°C (Japanese Patent Laid-Open Publication No. 78751/1994). Heating period is generally in the range of 10 - 60 minutes, preferably 20 - 40 minutes.

The aforementioned heating treatment specifically includes the blowing of steam, whereby the yeast is heated to a temperature from 90°C to the boiling point for about 30 minutes. The yeast cells are suspended into water or a buffer so as to give a concentration from about 1 to 20% by weight. The cell wall lytic enzyme is added in a concentration from 1 to 200 units, preferably from 5 to 100 units per 1 g of the yeast cell and reacted under slow stirring with the yeast cell at a pH value from 5.0 to 8.0 at a temperature of 20 to 50°C, preferably from 30 to 45°C for 1 to 2 hours. In this case, an alkali metal nitrite such as sodium nitrite may be added at a final concentration of 0.1 to 5 M to the reaction mixture to promote the reaction. After the reaction, agglomerated protein granules are recovered from the reaction mixture, and it may be recovered by the conventional means such as centrifugation or filtration through a membrane filter.

In this connection, the activity units of yeast cell wall lytic enzymes have been herein measured according to the method described in US Patent No. 3716452. The method described in US Patent No. 3716452 is outlined as follows.

To a mixture of 3 ml of a yeast suspension having a cell concentration of 5 mg/ml and 5 ml of a 1/15 M phosphate buffer at pH 7.5 are added 1 ml of a yeast cell wall lytic enzyme liquid and water to adjust the total volume to 10 ml, and the mixture are reacted at 25°C for 2 hours. After the reaction, the optical density (O.D.) at 800 nm is measured. As a control, in the same procedure as described above except that 1 ml of the enzyme liquid was replaced by 1 ml of water, the decreasing rate of the optical density at 800 nm is calculated from the following equation:

$$\text{Decreasing rate} = \frac{\text{O.D. of the control} - \text{O.D. of the enzyme reaction mixture}}{\text{O.D. of the control}} \times 100$$

An enzyme having a decreasing rate of 30% is defined as 1 unit of an enzyme activity. There is a proportional relationship between the decreasing rate and the enzyme concentration until the decreasing rate reaches 60%. In order to measure correctly the unit, the liquid enzyme should be appropriately diluted within a concentration in which the decreasing rate be in the range of 60% or less.

In order to further purify the agglomerated protein granules thus produced by the enzymatic decomposition reaction, the reaction mixture was adjusted to a pH value from 12.5 to 13.0 with an alkali such as sodium hydroxide to dissolve the agglomerated protein granules, and then adjusted to a pH value from 4 to 5 with an acid such as hydrochloric acid to cause isoelectric precipitation. Precipitates are collected by filtration, and desiccated to give a yeast protein.

[Yeast protein derived peptide composition]

In order to prepare a peptide composition from the yeast protein obtained as above, it is sufficient to hydrolyze the yeast protein with an acid or an alkali, and it is possible to get a composition having a desired molecular weight by selecting appropriately the conditions such as the amounts of an acid, an alkali or an enzyme, the reaction time, or the reaction temperature. While the desired molecular weight varies depending on the applications, the peptide composition having an average molecular weight from 200 to 5,000 is often selected as an ingredient for cosmetics. In this connection, the average molecular weight means herein a number average molecular weight calculated from the average number obtained from the ratio of the total nitrogen content of the yeast protein derived peptide composition to the amino nitrogen content and the average molecular weight of the amino acids in the yeast protein derived peptide obtained from the amino acid analysis.

In the acid hydrolysis of the yeast protein, an inorganic acid such as hydrochloric acid, sulfuric acid, phosphoric acid, nitric acid or hydrobromic acid, or an organic acid such as acetic acid or formic acid is used.

In the alkali hydrolysis of the yeast protein, an inorganic alkali such as sodium hydroxide, potassium hydroxide, lithium hydroxide, barium hydroxide, sodium carbonate, potassium carbonate or lithium carbonate is used.

In the enzymatic hydrolysis of the yeast protein, an acidic proteolytic enzyme such as pepsin, proctase A or proctase B, or neutral or alkaline proteolytic enzyme such as papain, bromelain, thermolysin, trypsin, pronase or chymotrypsin is used. It is also possible to use a neutral or alkaline proteolytic enzyme derived from bacteria such as subtilisin, Staphylococcus protease, or the like.

In either case of the hydrolysis, the hydrolysate of the yeast protein thus obtained is then purified, according to necessities, by gel filtration, ion exchange, ultrafiltration, dialysis, or electrodialysis after neutralization, and incorporated into hair cosmetics and skin care cosmetics directly in the form of a liquid or after pulverization.

[Properties of yeast protein derived peptide composition]

The molecular weight of the yeast protein derived peptide composition of the present invention can be easily controlled on its preparation to give a composition having a stable molecular weight with reproducibility, and the composition is light in color, low in smell and excellent in stability during storage.

The yeast protein derived peptide composition of the present invention has also excellent sorption propreties to hair and skin and an excellent moisturizing effect to afford gloss and moistness to hair and skin, and an effect for protecting hair and skin.

The properties of these yeast protein derived peptide compositions of the present invention will become clear from the analysis of the yeast protein derived peptide composition, the results of stability test during storage, sorption test to hair, or a test for confirming the increase of tensile strength of hair in Examples, or the results of evaluation in Application Examples.

[Uses of the yeast protein derived peptide composition]

The yeast protein derived peptide composition is mainly used as a component of cosmetics, and the cosmetics having the yeast protein derived peptide composition incorporated therein include hair cosmetics such as a shampoo, a hair rinse, a split hair coat, agents No. 1 and No. 2 for permanent waving, a hair cream, a hair conditioner, a set lotion, a hair color, a hair treatment, a liquid hair conditioner, a hair pack, and a hair care and hair growing agent, a toilet lotion, an after shaving lotion, a shaving foam, a variety of creams such as a vanishing cream, a cleansing cream, an emollient cream, a moisturizing cream, and a hand cream, a hair removing agent, a face pack, an emulsion, a body shampoo, a face washing agent, various soaps, a make-up product, a sunscreen product, and the like.

The yeast protein derived peptide composition is preferably incorporated into cosmetics in an amount of 0.1 to 30% by weight. That is to say, if the yeast protein derived peptide composition is incorporated in an amount less than that described above, the properties of yeast protein derived peptide composition cannot be exhibited sufficiently. On the other hand, if the yeast protein derived peptide composition is incorporated in an amount more than that described above, the effect of the yeast protein derived peptide composition is not increased in proportion to the concentration of it, and the feeling of the cosmetic may be deteriorated by the presence of excessive amount of the composition.

The components incorporated in the cosmetics in combination with the yeast protein derived peptide composition include for example alkyl sulfates such as sodium lauryl sulfate and ethanolamine lauryl sulfate, polyoxyethylene alkyl ether sulfates such as triethanolamine polyoxyethylene (2EO) lauryl ether sulfate, (wherein EO represents ethylene oxide, and the number in front of EO denotes the mole number of ethylene oxide added), and sodium polyoxyethylene (3EO) alkyl (having 11 - 15 carbon atoms alone or as a mixture of the two or more) ether sulfate, alkylbenzene sulfonates

4

such as sodium laurylbenzene sulfonate and triethanolamine laurylbenzene sulfonate, polyoxyethylene alkyl ether acetates such as polyoxyethylene (3EO) tridecyl ether, N-acyl amino acid salts such as sodium coconut oil fatty acid-L-glutamate and sodium coconut oil fatty acid sarcosinate, acylated protein hydrolysates that is the acylation product of a protein hydrolysate derived from animals or plants such as collagen, keratin, fibroin, casein, soybean, wheat or corn with a $C_8$ - $C_{20}$ fatty acid, anionic surface active agents such as sodium hydrogenated coconut oil fatty acid glycerol sulfate, polyoxyethylene alkyl (having 12 - 15 carbon atoms) ether phosphate (8 - 10EO), sodium polyoxyethylene cetyl ether phosphate, sodium coconut oil fatty acid methyltaurate and sodium coconut oil fatty acid isethionate, cationic surface active agents such as distearyldimethyl-ammonium chloride and stearyltrimethylammonium chloride, amphoteric surface active agents such as 2-alkyl (having 12 - 15 carbon atoms)-N-carboxylmethyl-N-hydroxyethyl-imidazolinium betaine, sodium undecylhydroxyethylimidazolinium betaine, an N-coconut oil fatty acid acyl-L-arginine ethyl-DL-pyrrolydonecarboxylic acid salt, coconut oil fatty acid amide propylbetaine and N-alkyl (having 12 - 18 carbon atoms) dimethylaminoacetic acid betaine, nonionic surface active agents such as polyoxyethylene alkyl (having 12 - 14 carbon atoms) ether (7EO), polyoxyethylene oleyl ether, polyoxyethylene nonyl phenyl ether, alkyl (having 4 - 20 carbon atoms) glucoside and alkyl polyglycoside, synthetic polymers such as a cationic polymer, an amphoteric polymer and an anionic polymer including cationized cellulose and cationized hydroxyethylcellulose, thickeners such as isostearic acid diethanolamide and lauric acid diethanolamide, extracts from animals and plants, polysaccharides or derivatives thereof, linear or cyclic methylpolysiloxane, methylphenyl polysiloxane, an amino-denatured silicone oil, humectants such as propylene glycol, 1,3-butylene glycol and glycerol, lower alcohols such as methanol, ethanol and propyl alcohol, amino acids such as sodium L-aspartate, DL-alanine, glycine, L-arginine and L-systeine. The other components can be appropriately added within the concentration that the effect of the present invention will not be impaired.

[Hair treatment agent having the yeast protein derived peptide composition incorporated therein]

In conventional hair treatment agents such as a hair rinse, a hair conditioner or a hair treatment, the combination of a cationic surface active agent and silicone has mainly been used to soften hair, improve the combability of hair and prevent static build-up with the cationic surface active agent, and to smooth the surface of hair, afford gloss to hair and prevent split hair due to combing.

The above described method, while it improves only the surface physical properties, cannot prevent the elution of protein from hair, tends to damage hair, and decreases moisture retention to cause dry hair. Thus, a protein hydrolysate has been added to the above described composition, but the conventional protein hydrolysate is not completely satisfactory in the point of its sorption property to hair.

The yeast protein derived peptide composition according to the present invention is excellent in sorption properties to hair as well as protecting effect and moisturizing effect on hair as compared with the currently used peptides for cosmetics. If the yeast protein derived peptide composition is incorporated in a hair treatment agent, the combability of hair is improved, the generation of split hair is prevented, and gloss and moistness is given to hair, so that the dry feeling of hair after washing is settled.

That is to say, while the yeast protein derived peptide composition protects hair, affords gloss and moistness to hair, and penetrates into hair to improve the moisture retention of hair for maintaining the moistness and gloss of hair for a long time, the composition also prevents the excessive adsorption of a cationic surface active agent onto hair and thus prevents the damage of hair due to the excessive adsorption of the cationic surface active agent.

The yeast protein derived peptide composition is preferably incorporated into a hair treatment agent in an amount (content in the hair treatment agent) from 0.05 to 10% by weight, particularly from 0.5 to 5% by weight. That is to say, if the yeast protein derived peptide composition is incorporated in an amount less than that described above, the protecting effect and moisturizing effect on hair as well as the effect for preventing the excessive adsorption of a cationic surface active agent are not exhibited sufficiently. On the other hand, if the yeast protein derived peptide composition is incorporated in an amount more than that described above, the effect of the yeast protein derived peptide composition is not increased in proportion to the concentration of it.

Also, in a treatment agent used for the treatment with a polypeptide (PPT) treatment mainly practiced during the intermediate step of the permanent waving treatment or the hair dyeing treatment, a protein hydrolysate is incorporated in a high concentration from 20 to 50% by weight. This was because the treatments such as permanent waving or hair dyeing involved the chemical reaction with hair and thus damaged hair seriously, and the PPT treatment agent was rinsed off after its application for a short time, so that the protein hydrolysate was required to be incorporated in a high concentration for increasing the effect of recovering hair from such damages. However, the application of the protein hydrolysate in such a high concentration will increase the price of the product, so that it is desired to develop a protein hydrolysate which is excellent in sorption properties to hair and can be applied only a small amount as compared with the conventional protein hydrolysate.

The yeast protein derived peptide composition of the present invention is excellent in sorption properties to hair as compared with protein hydrolysates which are generally used in PPT treatment agents, so that it is useful for the recovery from the damage of hair which have been particularly subjected to the permanent waving treatment or the hair dyeing treatment. That is to say, the yeast protein derived peptide composition penetrates into hair damaged by the permanent waving treatment or the hair dyeing treatment, increases the moisture retention of hair, and protect hair by forming a film on the hair owing to its film forming effect. The yeast protein derived peptide composition, which is soluble in water differently from silicone, will not affect deleteriously the waving in the permanent waving treatment or the hair dyeing effect in the hair dyeing treatment. Furthermore, the yeast protein derived peptide composition is excellent in the sorption properties to hair, so that it can be incorporated in a decreased amount into a PPT treatment agent and thus is excellent in a economical viewpoint.

The yeast protein derived peptide composition is preferably incorporated into a PPT treatment agent in an amount (content in the hair treatment agent) from 5 to 35% by weight, particularly from 10 to 30% by weight. That is to say, if the yeast protein derived peptide composition is incorporated in an amount less than that described above, the protecting effect and moisturizing effect on hair are not exhibited sufficiently. On the other hand, if the yeast protein derived peptide composition is incorporated in an amount more than that described above, the effect is not increased in proportion to the concentration of it.

[Shampoo having the yeast protein derived peptide composition incorporated therein]

In the conventional shampoos which have been generally used, anionic surface active agents such as a alkyl sulfate salt and a polyoxyethylene alkyl sulfate salt, nonionic surface active agents such as a polyoxyethylene alkyl ether and a fatty acid alkylolamide, and amphoteric surface active agents such as an alkyl betaine and an alkylamine oxide are incorporated solely or as a mixture thereof as a main component, and shampoos having a cationic surface active agent incorporated therein has been also proposed for affording moist feeling to hair.

However, if hair washing is conducted with a shampoo having these surface active agents incorporated therein, sebum, the other oily materials and proteins on the surface of hair will be rinsed off excessively. As a result, gloss or moistness are lost from hair, and the feeling of hair is deleteriously affected to cause dry hair, and combability or brushing property of hair are also deleteriously affected to cause split hair or broken hair.

Thus, it has been carried out to add a protein hydrolysate to a shampoo, to sorb it into hair, thus to feed the proteinaceous component to hair, so that gloss and moistness are afforded to hair, and split hair and broken hair are prevented by its film forming effect. However, the conventional protein hydrolysates are weak in sorption force and are rinsed off easily by washing with water, so that these hydrolysates will not exhibit their effects in a small amount. Thus, it has been conducted to increase the amount incorporated or to use a cationized peptide having a cationic group introduced therein, but such modifications will increase the price.

To the contrary, the yeast protein derived peptide composition of the present invention is very excellent in sorption properties to hair, so that when it is incorporated into a shampoo, it sorbs onto hair, penetrates into hair, affords moistness to hair by the moisturizing effect. Furthermore, if the yeast protein derived peptide composition sorbed on the surface of hair, it prevents split hair, affords gloss to hair, and improve the combability by its film forming effect, so that broken hair by combing or brushing is prevent.

The yeast protein derived peptide composition is preferably incorporated into a shampoo in an amount (content in the shampoo) from 0.05 to 5% by weight, particularly from 0.2 to 2% by weight. That is to say, if the yeast protein derived peptide composition is incorporated in an amount less than that described above, the protecting effect and moisturizing effect on hair as well as the effect of preventing the excessive sorption of a cationic surface active agent are not exhibited sufficiently. On the other hand, if the yeast protein derived peptide composition is incorporated in an amount more than that described above, the feeling is likely to be impaired by the stickiness caused by the remaining yeast protein derived peptide composition on the hair.

[Agent No. 1 for permanent waving having the yeast protein derived peptide composition incorporated therein]

The conventional permanent waving solution comprises the agent No. 1 of an aqueous solution containing as a main component a reducing agent such as thioglycolic acid or cysteine to which an basic material such as ammonia, monoethanolamine or triethanolamine is added to adjust pH 8 - 10, and the agent No. 2 of an aqueous solution of an oxidizing agent such as sodium bromate or hydrogen peroxide.

The mechanism of waving hair with the permanent waving solution comprises applying the agent No.1 to hair, winding the hair on rods so as to carl the hair, breaking the disulfide bond of cystine contained in keratin protein in hair with the agent No.1 to form mercapto groups, and then oxidizing the mercapto groups with the agent No. 2 to form a disulfide bond at different positions for fixing the waving.

With the permanent waving solution, the disulfide bond broken by the reduction with the agent No. 1 will not always restore the disulfide bond completely by the subsequent oxidation with the agent No. 2, and side-reactions are caused including a portion of the mercapto groups generated from cystine by the treatment with the agent No. 1 which is subjected to excessive oxidation with the agent No. 2 or is reacted with the mercapto group of thioglycolic acid or cysteine remaining in hair to form a disulfide bond. A portion of the keratin protein in hair is thus eluted, and the remaining portion in hair is also impaired by physical or chemical changes. As a result, not only a sense of incongruity or dry feeling is afforded to hair, but also hair is damaged seriously.

It has been therefore tried to incorporate a protein hydrolysate or a high molecular weight polymer such as silicone into a permanent waving solution to decrease the sense of incongruity of hair after the permanent waving treatment and recovering the impaired hair. However, the conventional protein hydrolysate is weak in sorption properties and thus rinsed off during shampooing to get only insufficient effect, and the high molecular weight polymer such as silicone forms a film on hair and thus descreases surface dry feeling of hair but has drawbacks that such a polymer has no drastic recovering effect on the damage of hair due to the elution of proteins from the hair and that the hair is difficult to be permanent-waved due to the presence of the film. Thus, it is desired to develop a peptide which does not affect the permanent waving treatment and is excellent in sorption properties.

The yeast protein derived peptide composition according to the present invention is very excellent in sorption properties to hair, so that it sorbs to hair, penetrates into the hair to which the permanent waving treatment has been subjected to exhibit the moisturizing effect, and retains the moistness of hair for a long period. The yeast protein derived peptide composition sorbed to the hair is excellent in sorption properties, thus hard to be rinsed off from the hair by mild rinsing, remains on the hair to suppress the excessive oxidation or the side-reactions of a mercapto group in the hair with thioglycolic acid and to decrease the damage of the hair. Furthermore, the yeast protein derived peptide composition of the present invention, different from the synthetic high molecular weight material such as silicone, has a similar structure to that of a keratin protein constituting hair, the hair can be always subjected to the permanent waving treatment in the presence of the yeast protein derived peptide composition.

Furthermore, the yeast protein derived peptide composition of the present invention prevent the excessive sorption of a cationic surface active agent, and thus prevent the damage of hair due to the excessive sorption of the cationic surface active agent. That is to say, among the agent No. 1 for the permanent waving, there is included the one having a cationic surface active agent incorporated therein for softening hair and making the hair permanent-waved easily. When the agent No. 1 for the permanent waving in which the cationic surface active agent has been incorporated is used, the yeast protein derived peptide composition of the present invention prevent forms a film on hair to prevent the excessive sorption of the cationic surface active agent onto the hair, so that the damage of the hair is prevented due to the excessive sorption of the cationic surface active agent.

The yeast protein derived peptide composition is preferably incorporated into the agent No. 1 for the permanent waving in an amount (content in the agent No. 1 for permanent waving) from 0.1 to 10% by weight, particularly from 0.5 to 5% by weight. That is to say, if the yeast protein derived peptide composition is incorporated into the agent No. 1 for the permanent waving in an amount less than that described above, the protecting effect and moisturizing effect on hair are not exhibited sufficiently. On the other hand, if the yeast protein derived peptide composition is incorporated into the agent No. 1 for the permanent waving in an amount more than that described above, the effect of the yeast protein derived peptide composition is not increased in proportion to the concentration of it, and the feeling of hair is likely to be impaired by the stickiness caused by the remaining yeast protein derived peptide composition on the hair.

[Hair dyeing agent having the yeast protein derived peptide composition incorporated therein]

In the permanent hair dyeing agent, oxidizing hair dyeing agents comprising the agent No. 1 containing an oxidizing dye and the agent No. 2 containing an oxidizing agent such as hydrogen peroxide have been extensively used. The oxidizing hair dyeing agents, in which an alkali agent is generally incorporated at a high pH of 10 or more to promote the uniform penetration of the oxidizing dye, however, has a drawback that it tends to damage hair and has an irritation, and that the proteinous components in the hair tend to be eluted.

Therefore, there have been also used acidic hair dyeing agents free of alkali agents, but the acidic hair dyeing agents also involve the oxidation step for the breaching treatment during hair dyeing and thus has the drawbacks that the hair tends to be damaged and loses gloss, and that the combability becomes worse.

In many of the temporary hair dyeing agents, hair dyeing is performed by adsorbing the dye on cuticula pili. Thus, the concentration of the dye is increased, or a large amount of a spreading agent or a polymeric resin is added in order to enhance the dyeing effect, so that the temporary hair dyeing agents have the drawbacks that the hair after the hair dyeing treatment is hardened, the combability becomes worse and hair tends to be damaged by combing.

More recently, there have been increasingly used hair dyeing agents of an acidic shampoo type which is free of an alkali agent. In these agents, a penetrating agent such as coconut oil fatty acid diethanolamide, polyoxyethylene nonyl phenyl ether, or benzyl alcohol is incorporated, so that these agents have the drawbacks that the impairment of hair and the elution of proteinaceous components tend to be caused, the hair after hair dyeing becomes dry and the combability becomes worse.

There has been thus proposed the incorporation of silicone or a protein hydrolysate into a hair dyeing agent for the purpose of solving the problems. Silicone, which forms a film on the surface of hair, improves the combability, but it does not settle the damage of hair due to the elution of proteinaceous components, and it additionally has a drawback that a dye is difficult to be fixed on the surface of hair due to the presence of the silicone film. The conventional protein hydrolysate is water-soluble and weak in sorbing force, so that the protein hydrolysate adsorbed on the surface of hair is easily rinsed off during the rinsing step of the hair dyeing treatment.

The yeast protein derived peptide composition according to the present invention is very excellent in the sorption properties to hair causes the elution of proteinaceous components due to rinsing, so that it can form a film on the surface of hair to prevent the impairment, afford gloss and moistness to hair, and penetrate into hair to retain the moistness and gloss of hair for a long period.

The yeast protein derived peptide composition is preferably incorporated into the hair dyeing agent in an amount (content in the hair dyeing agent) from 0.1 to 10% by weight, particularly from 1 to 5% by weight. That is to say, if the yeast protein derived peptide composition is incorporated into the hair dyeing agent in an amount less than that described above, the protecting effect and moisturizing effect on hair are not exhibited sufficiently. On the other hand, if the yeast protein derived peptide composition is incorporated into the hair dyeing agent in an amount more than that described above, the hair dyeing agent has a drawback that the feeling of hair is likely to be impaired by the stickiness caused by the remaining yeast protein derived peptide composition on the hair.

[Skin washing agent having the yeast protein derived peptide composition incorporated therein]

In skin washing agents such as a body shampoo and a facial washing agent, an anionic surface active agent having a relatively low irritation such as an amino acid type anionic surface active agent or an acylated derivative of a protein hydrolysate has been used as a main component. However, if washing is conducted with a skin washing agent having these surface active agents incorporated therein, sebum, the other oily materials and proteins will be rinsed off excessively. As a result, these skin washing agents thus have the drawback of a sense of incongruity such as drying and roughening of skin after washing.

While a protein hydrolysate has been thus incorporated into the washing agent, the conventional protein hydrolysate is sorbed on skin too weak to get a sufficient effect in the present state.

The yeast protein derived peptide composition according to the present invention is excellent also in the sorption propreties to skin, and washing of skin with a skin washing agent having the yeast protein derived peptide composition incorporated therein eliminates the feeling of drying and roughening the skin and affords the feeling of moistness to the skin.

The yeast protein derived peptide composition is preferably incorporated into the skin washing agent in an amount (content in the skin washing agent) from 0.1 to 10% by weight, particularly from 1 to 5% by weight. That is to say, if the yeast protein derived peptide composition is incorporated into the skin washing agent in an amount less than that described above, the moisturizing effect on skin is not exhibited sufficiently. On the other hand, if the yeast protein derived peptide composition is incorporated into the skin washing agent in an amount more than that described above, the feeling of skin is likely to be impaired by the stickiness caused by the remaining yeast protein derived peptide composition on the skin.

Examples

The present invention is specifically described with reference to Examples and Application Examples. In this connection, the present invention is not limited to these Examples and Application Examples.

First of all, the preparation of the yeast protein derived peptide composition according to the present invention is described in Examples 1 - 4, and the preparation of the yeast protein derived peptide composition according to the conventional method is described in Comparative Examples 1 - 4. In each of these Examples 1 - 4 and Comparative Examples 1 - 4, the preparation of the yeast protein derived peptide composition was carried out twice in order to confirm the reproducibility of the preparation of the yeast protein derived peptide composition. In this connection, the percentage representing the concentration of a solution means % by weight.

Example 1

Viable yeast cells belonging to the Candida genus (corresponding to 1 kg of the dry weight) were suspended into water and diluted with water to a total volume of 15 liters, and the suspension was heated with stirring at 95°C for 30 minutes. After cooling the suspension to room temperature, the cells were collected by centrifugation and washed with water. The cells were suspended again into 15 liters of water, 25,000 units of a yeast cell wall lytic enzyme produced by Arthrobacter luteus was added to the suspension, and the mixture was subjected to reaction with stirring at 35°C for 4 hours. The reaction mixture was centrifuged to collect the agglomerated protein granules, which were washed with water and lyophilized to give 390 g of a yeast protein as a powder. The powdery yeast protein has the total nitrogen content of 11.27%, and the protein content calculated from the total nitrogen content was 80%.

A 200 g portion of the yeast protein thus obtained was suspended into 1 liter of water, 40 g of sodium hydroxide was added, and the mixture was hydrolyzed with stirring at 70°C for 4 hours. The resulting hydrolysate was filtered through No. 2 filter paper (manufactured by TOYO FILTER PAPER K.K.) to remove insolubles, and the filtrate was diluted with about 300 ml of 6N hydrochloric acid to pH 3. The resulting insolubles were removed by filtration through No. 2 filter paper, and the filtrate was adjusted to pH 5.5 with a 20% aqueous sodium hydroxide solution and desalted with an electrodialyzer. After desalting, the desalted solution was concentrated to a 25% solution, adjusted to pH5.8 with a 20% aqueous sodium hydroxide solution, filtered through No. 5 filter paper (manufactured by TOYO FILTER PAPER K.K.), and further filtered aseptically through a membrane filter having a pore size of 0.45 $\mu$m to give a 25% aqueous solution of the yeast protein derived peptide composition. The yield and analysis of the yeast protein derived peptide composition are listed below after the description of Comparative Examples.

Example 2

A 200 g portion of the yeast protein obtained in the same manner as in Example 1 was suspended in 1 liter of water, and adjusted to pH 7 with a 20% aqueous sodium hydroxide solution. To the suspension was added 100 mg of trypsin [manufactured by NOVO INDUSTRY JAPAN K.K., PTN 3.0S (3.3 ANSON units)], and the mixture was subjected to decomposition with stirring at 50°C for 12 hours. Next, 10 g of sodium hydroxide was added, and the mixture was hydrolyzed with stirring at 65°C for 2 hours. The hydrolysate thus obtained was filtered through No. 2 filter paper to remove insolubles, and the filtrate was diluted with 180 g of 6N hydrochloric acid to pH 3. The resulting insolubles were removed by filtration through No. 2 filter paper, and the filtrate was adjusted to pH 5.8 with a 20% aqueous sodium hydroxide solution and desalted with an electrodialyzer. After desalting, the desalted solution was concentrated to a 25% solution, adjusted to pH5.8 with a 20% aqueous sodium hydroxide solution, filtered through No. 5 filter paper, and further filtered aseptically through a membrane filter having a pore size of 0.45 $\mu$m to give a 25% aqueous solution of the yeast protein derived peptide composition.

Example 3

A 200 g portion of the yeast protein obtained in the same manner as in Example 1 was suspended in 1 liter of water, and 10 g of sodium hydroxide was added. Subtilisin [100 mg; manufactured by NAGASE SANGYO K.K., bulk of BIOPLASE (790,000 units)] was added, and the mixture was hydrolyzed with stirring at 50°C for 12 hours. After the enzymatic hydrolysis was completed, 16 g of sodium hydroxide was added, and the mixture was subjected to alkaline hydrolysis with stirring at 65°C for 4 hours. The hydrolysate thus obtained was filtered through No. 2 filter paper to remove insolubles, and the filtrate was diluted with about 240 ml of 6N hydrochloric acid to pH 3. The resulting insolubles were removed by filtration through No. 2 filter paper, and the and the filtrate was adjusted to pH 5.8 with a 20% aqueous sodium hydroxide solution and desalted with an electrodialyzer. After desalting, the desalted solution was concentrated to a 25% solution, adjusted to pH5.8 with a 20% aqueous sodium hydroxide solution, filtered through No. 5 filter paper, and further filtered aseptically through a membrane filter having a pore size of 0.45 $\mu$m to give a 25% aqueous solution of the yeast protein derived peptide composition.

Example 4

To 200 g of the yeast protein obtained in the same manner as in Example 1 was added 600 go of 18% hydrochloric acid, and the mixture was hydrolyzed with stirring at 70°C for 5 hours. Ammonia gas was bubbled into the hydrolysate thus obtained to adjust the mixture to pH 4 and to deposit insolubles. The insolubles were removed by filtration through No. 2 filter paper, and the filtrate was again adjusted to pH about 10 by bubbling ammonia gas. The mixture was concentrated under reduced pressure to remove the excessive ammonia gas. The concentrate was left standing for 12 hours to deposit insolubles. The insolubles were removed by filtration through No. 2 filter paper, and the filtrate was adjusted to pH with hydrochloric acid and then desalted with an electrodialyzer. After desalting, the desalted solution was concentrated to a 25% solution, adjusted to pH5.8 with a 20% aqueous sodium hydroxide solution, filtered through No. 5 filter paper, and further filtered aseptically through a membrane filter having a pore size of 0.45 $\mu$m to give a 25% aqueous solution of the yeast protein derived peptide composition.

Comparative Example 1

Viable yeast cells belonging to the Candida genus (corresponding to 1 kg of the dry weight) were suspended into water and diluted with water to a total volume of 10 liters, and the suspension was subjected to homogenization treatment under a pressure of 800 kg/cm$^2$. To the homogenized suspension was added 1 liter of a 1N aqueous sodium hydroxide solution, and the mixture was stirred at 30°C for 30 minutes to extract protein. Next, the protein extract was separated from the residue by centrifugation. To the extract was added 6N hydrochloric acid to adjust the mixture to pH 4.5. After isoelectric precipitation, the mixture was centrifuged at 10,000 rpm for 10 minutes to collect precipitates, which was spray dried to give 280 g of the yeast protein as a powder. The powdery yeast protein has the total nitrogen content of 9.75%, and the protein content calculated from the total nitrogen content was 60.9%.

A 200 g portion of the yeast protein thus obtained was subjected to hydrolysis and purification in the same procedures as in Example 1 to give a 25% aqueous solution of the yeast protein derived peptide composition.

Comparative Example 2

A 200 g portion of the yeast protein obtained in the same manner as in Comparative Example 1 was subjected to hydrolysis and purification in the same procedures as in Example 2 to give a 25% aqueous solution of the yeast protein derived peptide composition.

Comparative Example 3

A 200 g portion of the yeast protein obtained in the same manner as in Comparative Example 1 was subjected to hydrolysis and purification in the same procedures as in Example 3 to give a 25% aqueous solution of the yeast protein derived peptide composition.

Comparative Example 4

A 200 g portion of the yeast protein obtained in the same manner as in Comparative Example 1 was subjected to hydrolysis and purification in the same procedures as in Example 4 to give a 25% aqueous solution of the yeast protein derived peptide composition.

Table 1 shows the analytical results of the yeast protein derived peptide compositions in Examples 1 and 2, Table 2 shows the analytical results of the yeast protein derived peptide compositions in Examples 3 and 4, Table 3 shows the analytical results of the yeast protein derived peptide compositions in Comparative Examples 1 and 2, and Table 4 shows the analytical results of the yeast protein derived peptide compositions in Comparative Examples 3 and 4.

The total nitrogen content of the yeast protein derived peptide composition in the tables is the value measured with a total carbon-total nitrogen analyzer, and the yield is the value obtained by comparing with the total nitrogen content in the yeast protein and indicates the overall yield starting from the yeast protein. The composition of amino acids are obtained from the measurements with an amino acid autoanalyzer, and the hue is measured according to the Gardner method. The average molecular weight is the number average molecular weight obtained by the method described above.

Table 1

|  | Example 1 | | Example 2 | |
| --- | --- | --- | --- | --- |
|  | First | Second | First | Second |
| Amount (g) | 280 | 292 | 234 | 242 |
| Total nitrogen content (%) | 2.436 | 2.452 | 2.555 | 2.651 |
| Yield (%) | 30.3 | 31.8 | 26.5 | 28.5 |
| Average molecular weight | 1,606 | 1,598 | 906 | 910 |
| Composition of amino acid (molar %) |  |  |  |  |
| Basic | 14.2 | 14.1 | 17.1 | 17.6 |
| Acidic | 24.9 | 24.8 | 23.6 | 23.7 |
| Neutral | 60.9 | 61.1 | 59.3 | 58.7 |
| Hue | 10 | 10 | 10 | 10 |

Table 2

|  | Example 3 | | Example 4 | |
|---|---|---|---|---|
|  | First | Second | First | Second |
| Amount (g) | 320 | 331 | 268 | 273 |
| Total nitrogen content (%) | 2.954 | 2.874 | 3.605 | 3.600 |
| Yield (%) | 41.9 | 42.2 | 42.8 | 43.6 |
| Average molecular weight | 593 | 598 | 394 | 400 |
| Composition of amino acid (molar %) |  |  |  |  |
| Basic | 12.6 | 12.8 | 14.7 | 14.9 |
| Acidic | 24.2 | 24.4 | 21.3 | 22.0 |
| Neutral | 63.2 | 62.8 | 64.0 | 62.5 |
| Hue | 11 | 11 | 15 | 14 |

Table 3

|  | Comparative Example 1 | | Comparative Example 2 | |
|---|---|---|---|---|
|  | First | Second | First | Second |
| Amount (g) | 92 | 87 | 154 | 127 |
| Total nitrogen content (%) | 2.757 | 2.522 | 3.227 | 2.801 |
| Yield (%) | 11.5 | 10.8 | 19.9 | 21.4 |
| Average molecular weight | 2,084 | 1,711 | 825 | 677 |
| Composition of amino acid (molar %) |  |  |  |  |
| Basic | 14.4 | 13.1 | 14.0 | 15.2 |
| Acidic | 22.6 | 24.8 | 23.1 | 21.6 |
| Neutral | 63.0 | 62.1 | 62.9 | 63.2 |
| Hue | 14 | 15 | $\geq 18$ | $\geq 18$ |

Table 4

| | Comparative Example 3 | | Comparative Example 4 | |
|---|---|---|---|---|
| | First | Second | First | Second |
| Amount (g) | 217 | 202 | 197 | 201 |
| Total nitrogen content (%) | 3.073 | 2.890 | 3.502 | 3.414 |
| Yield (%) | 34.3 | 29.9 | 35.4 | 35.2 |
| Average molecular weight | 495 | 401 | 392 | 288 |
| Composition of amino acid (molar %) | | | | |
| Basic | 13.7 | 12.5 | 14.9 | 13.4 |
| Acidic | 22.4 | 23.2 | 20.5 | 22.7 |
| Neutral | 63.9 | 64.3 | 64.6 | 63.9 |
| Hue | 16 | 17 | $\geqq 18$ | $\geqq 18$ |

As described above, in each of Examples and Comparative Examples, the preparation of the yeast protein derived peptide composition was carried out twice in order to confirm the reproducibility of its preparation. Each table shows the analytical results the yeast protein derived peptide composition obtained in the first and second preparations in each of Examples and Comparative Examples.

As shown in Tables 1 and 2, large differences were not observed for the yield, average molecular weight, composition of amino acids and hue of the yeast protein derived peptide composition between the first and second preparations of the peptide in Examples 1 - 4, and thus the yeast protein derived peptide composition could be prepared with good reproducibility.

On the contrary, as shown in Tables 3 and 4, differences were observed for the analyses of the yeast protein derived peptide composition between the first and second preparations of the peptide in Comparative Examples 1 - 4, and thus indicated that the molecular weight of the yeast protein derived peptide composition was difficult to control according to the method in Comparative Examples 1 - 4.

When the protein hydrolysate is intended to put to practical use, it is important to maintain a constant average molecular weight for securing the effect. The yeast protein derived peptide composition according to the present invention is found to be suitable for practical use, as it has little difference in analytical results including the average molecular weight between the first and second peptide preparation.

Also, referring to hue, it is found that the yeast protein derived peptide compositions in Examples 1 - 4 are light in color as compared with those in Comparative Examples 1 - 4, and thus affect little the hue of products in practical use, for example in cosmetics.

[Storage stability of an aqueous solution of the yeast protein derived peptide composition]

A 25% aqueous solution of the yeast protein derived peptide composition prepared in Examples 1 - 4 and Comparative Examples 1 - 4 was stored in an incubator at 40°C for 60 days to check the change of hue and the generation of precipitates during the storage. The change of hue was examined by the Gardner method, and the amount of precipitates was evaluated on the basis of the following grades.

Evaluation grades

+++:     a very large amount of precipitates,
++:      a large amount of precipitates,
+:       a little amount of precipitates,
-:       no precipitates.

Table 5 shows the results of storage stability of the yeast protein derived peptide compositions in Examples 1 and 2, Table 6 shows the results of storage stability of the yeast protein derived peptide compositions in Examples 3 and 4, Table 7 shows the results of storage stability of the yeast protein derived peptide compositions in Comparative Examples 1 and 2, and Table 8 shows the results of storage stability of the yeast protein derived peptide compositions in Comparative Examples 3 and 4.

Table 5

| Yeast protein derived peptide composition | | Evaluation items | Storage period (day) | | | | |
|---|---|---|---|---|---|---|---|
| | | | 10 | 20 | 30 | 45 | 60 |
| Example 1 | First | Precipitates | - | - | - | - | - |
| | | Hue | 10 | 11 | 12 | 12 | 12 |
| | Second | Precipitates | - | - | - | - | - |
| | | Hue | 10 | 11 | 12 | 12 | 12 |
| Example 2 | First | Precipitates | - | - | - | - | - |
| | | Hue | 10 | 10 | 11 | 11 | 12 |
| | Second | Precipitates | - | - | - | - | - |
| | | Hue | 10 | 10 | 11 | 11 | 12 |

Table 6

| Yeast protein derived peptide composition | | Evaluation items | Storage period (day) | | | | |
|---|---|---|---|---|---|---|---|
| | | | 10 | 20 | 30 | 45 | 60 |
| Example 3 | First | Precipitates | - | - | - | - | - |
| | | Hue | 11 | 11 | 11 | 11 | 11 |
| | Second | Precipitates | - | - | - | - | - |
| | | Hue | 11 | 11 | 11 | 11 | 11 |
| Example 4 | First | Precipitates | - | - | - | - | - |
| | | Hue | 15 | 16 | 16 | 16 | 16 |
| | Second | Precipitates | - | - | - | - | - |
| | | Hue | 14 | 15 | 16 | 16 | 16 |

Table 7

| Yeast protein derived peptide composition | | Evaluation items | Storage period (day) | | | | |
|---|---|---|---|---|---|---|---|
| | | | 10 | 20 | 30 | 45 | 60 |
| Comparative Example 1 | First | Precipitates | + | ++ | +++ | +++ | +++ |
| | | Hue | 14 | 16 | 17 | >18 | >18 |
| | Second | Precipitates | - | + | ++ | +++ | +++ |
| | | Hue | 15 | 16 | 17 | >18 | >18 |
| Comparative Example 2 | First | Precipitates | - | + | ++ | ++ | ++ |
| | | Hue | >18 | >18 | >18 | >18 | >18 |
| | Second | Precipitates | + | ++ | +++ | +++ | +++ |
| | | Hue | >18 | >18 | >18 | >18 | >18 |

Table 8

| Yeast protein derived peptide composition | | Evaluation items | Storage period (day) | | | | |
|---|---|---|---|---|---|---|---|
| | | | 10 | 20 | 30 | 45 | 60 |
| Comparative Example 3 | First | Precipitates | - | + | ++ | +++ | +++ |
| | | Hue | 16 | 17 | >18 | >18 | >18 |
| | Second | Precipitates | - | + | + | ++ | ++ |
| | | Hue | 17 | >18 | >18 | >18 | >18 |
| Comparative Example 4 | First | Precipitates | - | - | + | + | + |
| | | Hue | >18 | >18 | >18 | >18 | >18 |
| | Second | Precipitates | - | - | + | + | ++ |
| | | Hue | >18 | >18 | >18 | >18 | >18 |

While no precipitates were observed, as shown in Tables 5 and 6, during storage for 60 days in the yeast protein derived peptide compositions of Examples 1 - 4, precipitates were observed during storage in the yeast protein derived peptide compositions of Comparative Examples 1 - 4 as shown in Tables 7 and 8. It is thus found that the yeast protein derived peptide compositions of Examples according to the present invention is more excellent in storage stability than those of Comparative Examples.

Moreover, it was found in the yeast protein derived peptide compositions of Comparative Examples 1 - 4 that two samples in each example have different number of days for generating precipitates for the first time and thus produce hydrolysates having a large difference between these molecular weight distributions.

Referring to the hue, it was indicated from Tables 7 - 8 that the hue changes largely during storage and turned brown with a hue of more than 18 on storage for 45 days. On the contrary, the yeast protein derived peptide compositions of Examples 1 - 4, while they turned dark a little during storage as shown in Tables 5 and 6, did not change their color on and after a certain point of time.

It is found from the facts described above that the yeast protein derived peptide compositions of the present invention have very excellent properties as compared with the conventional ones.

[Sorption test of the yeast protein derived peptide composition to hair]

The sorption of the yeast protein derived peptide composition to hair was tested according to the column circulating method in "The Method of Evaluating the Damages of Hair" described in Journal of SCCJ, Vol. 21, No. 2.

That is to say, hair (1.8 g) is filled in a column for liquid chromatography having a diameter of 7.5 mm and a length of 75 mm, and a test solution having a sample concentration of 2% is circulated at a flow rate of 2 ml/min through the column.

The sample concentration in the test solution after circulation was measured by subjecting the test solution to gel filtration analysis, and the amount of sample sorbed to 1 g of hair from the variation of the sample concentration in the test solution before and after circulation. In this connection, the decreased amount of the sample concentration due to the penetration into hair is corrected by circulating a test solution containing 2% polyoxyethylene glycol having an average molecular weight of 1,000 as a control under the same condition as above and regarding the decreased amount of the concentration of polyoxyethylene glycol as the amount sorbed to hair.

Sorption test 1

The yeast protein derived peptide composition (average molecular weight: 906) prepared in Example 2-1, the yeast protein derived peptide composition (average molecular weight: 825) prepared in Comparative Example 2-1, a silk derived peptide having an average molecular weight of 1,000 and a wheat derived peptide having an average molecular weight of 1,000 were used as samples. A 2% aqueous solution of each sample was prepared and used as a test solution, and the sorption to hair was examined by the method described above. The result is shown in Figure 1.

As shown in Figure 1, the sorption force of the yeast protein derived peptide composition of Example 2-1 was about 1.4 times of that of the yeast protein derived peptide composition of Comparative Example 2-1, about 3 times of the silk derived peptide, and about 2.5 times of the wheat protein derived peptide having a similar level of molecular weight.

Sorption test 2

The yeast protein derived peptide composition (average molecular weight: 598) prepared in Example 3-2, the yeast protein derived peptide composition (average molecular weight: 401) prepared in Comparative Example 3-2, a collagen derived peptide (average molecular weight: 450) and a soybean derived peptide (average molecular weight: 480) were employed to prepare a 2% aqueous solution of each sample, which was used as a test solution, and the sorption to hair was examined by the method described above. The result is shown in Figure 2.

As shown in Figure 2, the sorption force of the yeast protein derived peptide composition of Example 3-2 was about 1.4 times of that of the yeast protein derived peptide composition of Comparative Example 3-2, about 2.1 times of the collagen derived peptide, and about 1.7 times of the soybean protein derived peptide having a similar level of molecular weight.

Although the yeast protein derived peptide compositions of Examples 2-1 and 3-2 used in this test have a little higher molecular weight than that of the yeast protein derived peptide compositions of Comparative Examples 2-1 and 3-2, the yeast protein derived peptide compositions of Examples are apparently excellent in sorption properties as compared with the yeast protein derived peptide compositions of Comparative Examples, because it is generally considered that in peptides derived from the same protein a peptide having a lower molecular weight is superior in sorption properties to the one having a higher molecular weight.

[Test for confirming the enhancement of the tensile strength of hair by the yeast protein derived peptide composition]

The enhancement of the tensile strength of hair by the yeast protein derived peptide composition was confirmed according to the method in "The Method of Evaluating the Damages of Hair" described in Journal of SCCJ, Vol. 21, No. 2 described above.

Hair which had been once subjected to decoloration treatment to ensure almost constant strength of hair was employed in the test in order to keep the unevenness of the strength of hair for the test. That is to say, a tuft having a length of 10 cm and a weight of 1 g was impregnated in 10 g of a 1 : 1 mixture of a 10% aqueous hydrogen peroxide solution and a 10% aqueous ammonia solution for 30 minutes, rinsed with ion-exchanged water, and dried for the test.

A 5% aqueous solution of the yeast protein derived peptide composition of Example 4-2 (average molecular weight: 400) and a 5% aqueous solution of the yeast protein derived peptide composition of Comparative Example 4-1 (average molecular weight: 392) were used as a test solution, and the tuft which had been subjected to decoloration treatment as above was impregnated into one of the test solutions at 40°C for 5 minutes. After impregnation, the tuft was sufficiently rinsed with ion-exchanged water, and dried with a hair drier. After the procedure was repeated thrice, 30 hairs were removed from the tuft, and subjected to the tensile strength test.

In the tensile strength test, the longitudinal diameter and transversal diameter at the center (5 cm from the end) of hair were measured with a micrometer to calculate the cross section. The tensile strength at this point was next measured with a tensile strength tester (RHEOMETER, manufactured by FUDO KOGYO K.K.) to calculate the tensile strength per cross section.

As a control for comparison, an untreated tuft which had been subjected only to decoloration treatment and a 5% aqueous solution of collagen derived peptide or soybean derived peptide (both having an average molecular weight of about 450) were used, and the tensile strength of the hair which had been impregnated was measured in the same manner as above. The results are shown in Table 9.

Table 9

| | Tensile strength per cross section (kgf/mm²) | Enhancement of strength on the untreated hair (%) |
|---|---|---|
| Untreated hair (decolored hair) | 28.4 | - |
| Yeast protein derived peptide composition in Example 4-2 | 32.4 | 14.08 |
| Yeast protein derived peptide composition in Comparative Example 4-1 | 31.6 | 11.27 |
| Collagen derived peptide | 30.4 | 7.04 |
| Soybean derived peptide | 30.7 | 8.10 |

As shown in Table 9, the hair treated with the yeast protein derived peptide composition of Example 4-2 had an enhanced strength of 14% over the untreated hair. On the other hand, the hair treated with the yeast protein derived peptide composition of Comparative Example 4-1 had an enhanced strength of 11%, so that the yeast protein derived peptide composition of Example 4-2 had enhanced the strength about 1.2 times as compared with the yeast protein derived peptide composition of Comparative Example 4-1.

Compared with hair treated with the other peptides, the hair treated with the yeast protein derived peptide composition of Example 4-2 had an enhanced strength of about 2 times over the hair treated with the collagen derived peptide and of about 1.7 times over the hair treated with the soybean derived peptide. That is, the yeast protein derived peptide composition of the present invention apparently increased the strength of hair by sorbing to hair.

[Application Examples]

Application Examples in which the yeast protein derived peptide composition of the present invention has been incorporated into a variety of cosmetics are described below.

Application Example 1

The yeast protein derived peptide composition (average molecular weight: 1,606) obtained in Example 1-1 was incorporated to prepare a hair treatment cream (Practical Product 1) shown in Table 10. As the controls for comparison, a hair treatment cream in which a collagen derived peptide had been incorporated in place of the yeast protein derived peptide composition in Practical Product 1 (Comparative Product 1) and a hair treatment cream having neither of them incorporated (Comparative Product 2) were prepared to examine the moistness, gloss and combability of hair to which each of the hair treatment creams was applied.

In this connection, the amounts of materials used for preparing the hair treatment creams without indication of the ingredient concentration in the parentheses behind the material names are those based on the pure material. The amount incorporated of the ingredients are based on % by weight. These definitions stand up also in the following application examples.

Table 10

| | Practical Product 1 | Comparative Product 1 | Comparative Product 2 |
|---|---|---|---|
| Yeast protein derived peptide composition in Example 1-1 (average molecular weight: 1,606)(25%) | 12.0 | 0 | 0 |
| Collagen derived peptide (average molecular weight: 2,000) (PROMOIS W-52, manufactured by SEIWA KASEI, 30%) | 0 | 10.0 | 0 |
| Diglyceryl diisostearate | 5.0 | 5.0 | 5.0 |
| Ethyelene glycol monostearate | 3.0 | 3.0 | 3.0 |
| Stearic acid diethylaminoethylamide | 1.0 | 1.0 | 1.0 |
| Behenyl alcohol | 6.0 | 6.0 | 6.0 |
| Aqueous phosphoric acid solution (30%) | 0.5 | 0.5 | 0.5 |
| Polyglycerol | 2.0 | 2.0 | 2.0 |
| p-hydroxy-benzoic acid ester-phenoxy ethanol mixture (SEISEPT G, manufactured by SEIWA KASEI) | 0.3 | 0.3 | 0.3 |
| Sterilized ion-exchanged water | to 100 | to 100 | to 100 |

A tuft having a weight of 1 g and a length of 15 cm was washed with a 1% aqueous polyoxyethylene phenyl ether solution and rinsed with hot water, and 1 g of either one of the hair treatment creams of Practical Product 1 and Comparative Product 1 - 2 was applied to the tuft. The tuft was left standing for 5 minutes, then rinsed with hot water, and dried with a hair drier.

After the procedure was repeated five times, the moistness, gloss and combability of hair were evaluated by 10 panelers including 6 women and 4 men on the basis of the following criterion for evaluation. The results of the evaluation are listed digitally in Table 11. Values shown in the table are the average values of the evaluations by 10 panelers.

Criterion of evaluation

Best:          2,
Secondary:     1,
Inferior:      0.

Table 11

|                      | Practical Product 1 | Comparative Product 1 | Comparative Product 2 |
|----------------------|---------------------|-----------------------|-----------------------|
| Hair after treatment: |                     |                       |                       |
| Moistness            | 2.0                 | 1.0                   | 0.0                   |
| Gloss                | 1.8                 | 1.2                   | 0.0                   |
| Combability          | 1.8                 | 1.2                   | 0.0                   |

As is apparent from the results shown in Table 11, Practical Product 1 having the yeast protein derived peptide composition of Example 1-1 incorporated therein was superior to Comparative Product 1 having the collagen derived peptide incorporated therein or Comparative Product 2 having no peptide incorporated therein in relation to the gloss, moistness and combability of hair after treatment.

Application Example 2

A PPT treatment agent (Practical Product 2) shown in Table 12 was prepared by incorporating the yeast protein derived peptide composition (average molecular weight: 598) obtained in Example 3-2 therein. As the controls for comparison, a PPT treatment agent (Comparative Product 3) having a collagen derived peptide (average molecular weight: 440) in place of the yeast protein derived peptide composition in Practical Product 2 incorporated therein and a PPT treatment agent (Comparative Product 4) having neither of these peptide derivatives incorporated therein were prepared, and tufts subjected to decoloration treatment were treated with these PPT treatment agents.

Table 12

|                                                                                                      | Practical Product 2 | Comparative Product 3 | Comparative Product 4 |
|------------------------------------------------------------------------------------------------------|---------------------|-----------------------|-----------------------|
| Yeast protein derived peptide composition in Example 3-2 (average molecular weight: 598) (25%)        | 80.0                | 0                     | 0                     |
| Collagen derived peptide (average molecular weight: 440) (PROMOIS W-32R, manufactured by SEIWA KASEI, 30%) | 0                   | 66.7                  | 0                     |
| Benzarconium chloride (50%)                                                                           | 5.0                 | 5.0                   | 5.0                   |
| Concentrated glycerol                                                                                 | 5.0                 | 5.0                   | 5.0                   |
| Ethanol (99%)                                                                                         | 5.0                 | 5.0                   | 5.0                   |
| Sterilized ion-exchanged water                                                                       | to 100              | to 100                | to 100                |

First of all, tufts used for the test were decolored as follows. A tuft having a weight of 1 g and a length of 15 cm was impregnated in 10 g of a 1 : 1 mixture of a 10% aqueous hydrogen peroxide solution and a 10% aqueous ammonia solution for 30 minutes, rinsed with ion-exchanged water, and dried for the test.

A 1 g portion of the PPT treatment agents in Practical Product 2 and Comparative Products 3 and 4 was applied uniformly to the tuft having subjected to the decoloration treatment, and the tuft was left standing for 5 minutes. The tuft was then washed with a peptide-free shampoo commercially available, rinsed with hot water, and dried. The moistness, gloss and combability of hair thus treated were evaluated by 10 panelers including 6 women and 4 men on the basis of the same criterion as in Application Example 1. 30 hairs were removed from the tuft, and the tensile strength was measured at the center (7.5 cm from the end) of hair to calculate the tensile strength per cross section. The results are shown in Table 9. Values in the table are the average values of the evaluations.

Table 13

|  | Practical Product 2 | Comparative Product 3 | Comparative Product 4 |
|---|---|---|---|
| Hair after treatment: |  |  |  |
| Moistness | 2.0 | 1.0 | 0.0 |
| Gloss | 2.0 | 1.0 | 0.0 |
| Combability | 2.0 | 1.0 | 0.0 |
| Tensile strength of hair per cross section (kgf/mm$^2$) | 33.1 | 30.8 | 28.2 |

As shown in Table 13, Practical Product 2 having the yeast protein derived peptide composition in Example 3-2 got a high score for all of the moistness, gloss and combability of hair. Also, in relation to the tensile strenght per cross section, Practical Product 2 having the yeast protein derived peptide composition in Example 3-2 had an enhanced strength of about 17% over the peptide-free Comparative Product 4 and about 2 times over Comparative Product 3 having the collagen derived peptide incorporated therein, and thus indicated that the yeast protein derived peptide composition had an excellent sorption properties to hair and an excellent effect of preventing the damage of hair.

Application Example 3

A shampoo (Practical Product 3) shown in Table 14 was prepared by incorporating the yeast protein derived peptide composition (average molecular weight: 906) obtained in Example 2-1 therein. As the controls for comparison, a shampoo (Comparative Product 5) having a fibroin derived peptide (average molecular weight: 1,000) in place of the yeast protein derived peptide composition in Practical Product 3 incorporated therein and a shampoo (Comparative Product 6) having neither of these peptide derivatives incorporated therein were prepared, and hair was washed with these shampoos.

Table 14

| | Practical Product 3 | Comparative Product 5 | Comparative Product 6 |
|---|---|---|---|
| Yeast protein derived peptide composition in Example 2-1 (average molecular weight: 906) (25%) | 5.0 | 0 | 0 |
| Fibroin derived peptide (average molecular weight: 1,000) (PROMOIS SILK-1000, manufactured by SEIWA KASEI, 30%) | 0 | 17.8 | 0 |
| Potassium salt of a condensate of a collagen derived peptide with a coconut oil fatty acid (PROMOIS ECP, manufactured by SEIWA KASEI, 35%) | 12.0 | 12.0 | 12.0 |
| Coconut oil fatty acid diethanolamide | 3.0 | 3.0 | 3.0 |
| Ethylene glycol monostearate | 0.5 | 0.5 | 0.5 |
| Mixture of a p-hydroxybenzoic acid ester and phenoxyethanol (SEISEPT G, manufactured by SEIWA KASEI) | 0.5 | 0.5 | 0.5 |
| Citric acid | to pH 6 | to pH 6 | to pH 6 |
| Sterilized ion-exchanged water | to 100 | to 100 | to 100 |

A tuft having a weight of 1 g and a length of 15 cm was washed with either one of the shampoos of Practical Product 3 and Comparative Products 5 and 6, rinsed with water and dried with a drier. After the procedure was repeated five times, the moistness, gloss, smoothness and combability of hair were evaluated by 10 panelers including 6 women and 4 men on the basis of the same criterion as in Application Example 1. The results are shown in Table 15.

Table 15

| | Practical Product 1 | Comparative Product 1 | Comparative Product 2 |
|---|---|---|---|
| Hair after treatment: | | | |
| Gloss | 2.0 | 1.0 | 0.0 |
| Moistness | 1.8 | 1.2 | 0.0 |
| Smoothness | 2.0 | 1.0 | 0.0 |
| Combability | 1.9 | 1.1 | 0.0 |

As is apparent from the results in Table 15, Practical Product 3 having the yeast protein derived peptide composition in Example 2-1 got a higher score for all of the gloss, moistness, smoothness and combability of shampooed hair over Comparative Product 5 having the fibroin derived peptide incorporated therein and Comparative Product 6 which was free of peptide.

Application Example 4

An agent No. 1 for permanent waving (Practical Product 4) shown in Table 16 was prepared by incorporating the yeast protein derived peptide composition (average molecular weight: 910) obtained in Example 2-2 therein. As the controls for comparison, an agent No. 1 for permanent waving (Comparative Product 7) having a wheat protein derived peptide (average molecular weight: 1,000) in place of the yeast protein derived peptide composition in Practical Product 4 incorporated therein and an agent No. 1 for permanent waving (Comparative Product 8) having neither of these peptide derivatives incorporated therein were prepared, and hair was subjected to permanent waving treatment with these agents for permanent waving.

Table 16

|  | Practical Product 4 | Comparative Product 7 | Comparative Product 8 |
|---|---|---|---|
| Yeast protein derived peptide composition in Example 2-2 (average molecular weight: 910) (25%) | 8.0 | 0 | 0 |
| Wheat protein derived peptide (average molecular weight: 1,000) (25%) | 0 | 8.0 | 0 |
| Ammonium thioglycolate (50%) | 10.0 | 10.0 | 10.0 |
| Polyethylene oxide (7EO) oleyl ether | 0.3 | 0.3 | 0.3 |
| Polyethylene oxide (20EO) oleyl ether | 0.8 | 0.8 | 0.8 |
| Monoethanolamine (80%) | 1.5 | 1.5 | 1.5 |
| EDTA·2Na | 0.1 | 0.1 | 0.1 |
| Perfume | q.s. | q.s. | q.s. |
| Aqueous ammonia (28%) | to pH 9.0-9.5 | to pH 9.0-9.5 | to pH 9.0-9.5 |
| Sterilized ion-exchanged water | to 100 | to 100 | to 100 |

A tuft having a weight of 1 g and a length of 15 cm was used as a tuft for test, and a 6% aqueous sodium bromate solution was used as the agent No. 2. The tuft was subjected to permanent waving treatment five times.

The pliability, gloss and moistness of hair after the permanent waving treatment were evaluated by 10 panelers on the basis of the same criterion as in Application Example 1. Moreover, the hair having been subjected to permanent waving treatment was hydrolyzed, and subjected to amino acid analysis to quantitatively determine cysteic acid produced by the permanent waving treatment. Furthermore, 30 hairs were removed from each of the tufts, and the tensile strength of these hairs was measured to calculate the tensile strength per cross section. The results are shown in Table 17.

Table 17

|  | Practical Product 4 | Comparative Product 7 | Comparative Product 8 |
|---|---|---|---|
| Hair after permanent waving treatment: |  |  |  |
| Pliability | 2.0 | 1.0 | 0.0 |
| Gloss | 1.9 | 1.1 | 0.0 |
| Moistness | 2.0 | 1.0 | 0.0 |
| Amount of cysteic acid in hair ($\mu$mole/g) | 34 | 40 | 61 |
| Tensile strength per cross section of hair (kgf/mm$^2$) | 32.2 | 30.0 | 27.9 |

As is apparent from the results in Table 17, Practical Product 4 having the yeast protein derived peptide composition in Example 2-2 got a higher score for all of the pliability, gloss and moistness of hair which had been subjected to permanent waving treatment over Comparative Product 7 having the wheat protein derived peptide incorporated therein and Comparative Product 8 which was free of peptide. Thus, it is indicated that the yeast protein derived peptide composition in Example 2-2 had an excellent sorption properties to hair, the yeast protein derived peptide composition affords gloss and moistness to hair.

Also, the amount of cysteic acid in hair to which Practical Product 4 having the yeast protein derived peptide composition incorporated therein was applied was lesser than that in hair to which Comparative Product 7 having the wheat protein derived peptide incorporated therein or Comparative Product 8 which was free of peptide, and hair to which Practical Product 4 was applied had a larger tensile strength per cross section of hair than the other hairs to which the other products were applied.

The amount of cysteic acid in hair indicates the degree of the damage of hair due to the permanent waving treatment, and thus the lesser amount of cysteic acid in hair to which Practical Product 4 having the yeast protein derived peptide composition incorporated therein was applied indicates that the yeast protein derived peptide composition prevented damage of hair during the permanent waving treatment. In addition, the higher tensile strength in the case of using Practical Product 4 indicates that the yeast protein derived peptide composition sorbs to hair, increases the tensile strength of hair, and prevents the damage of hair during the permanent waving treatment.

Application Example 5

An agent No. 1 for a hair dye (Practical Product 5) shown in Table 5 was prepared by incorporating the yeast protein derived peptide composition (average molecular weight: 593) obtained in Example 3-1. As the controls for comparison, an agent No. 1 for hair dye (Comparative Product 9) having a casein derived peptide (average molecular weight: 600) in place of the yeast protein derived peptide composition in Practical Product 5 incorporated therein and an agent No. 1 for a hair dye (Comparative Product 10) having neither of these peptide derivatives incorporated therein were prepared.

Table 18

|  | Practical Product 5 | Comparative Product 9 | Comparative Product 10 |
|---|---|---|---|
| Yeast protein derived peptide composition in Example 3-1(average molecular weight: 593) (25%) | 4.0 | 0 | 0 |
| Casein protein derived peptide(average molecular weight: 600) (PROMOIS MILK, manufactured by SEIWA KASEI, 30%) | 0 | 3.3 | 0 |
| p-phenylene diamine | 0.8 | 0.8 | 0.8 |
| p-aminophenol | 0.2 | 0.2 | 0.2 |
| o-aminophenol | 1.0 | 1.0 | 1.0 |
| Resorcinol | 1.6 | 1.6 | 1.6 |
| Isopropanol | 10.0 | 10.0 | 10.0 |
| Propylene glycol | 12.0 | 12.0 | 12.0 |
| Oleic acid | 20.0 | 20.0 | 20.0 |
| Bis-2-hydroxyethylsorbitanamine | 9.0 | 9.0 | 9.0 |
| Hydroxyethylstearylamide | 6.0 | 6.0 | 6.0 |
| Aqueous ammonia (28%) | 10.0 | 10.0 | 10.0 |
| Sodium nitrite | 0.5 | 0.5 | 0.5 |
| EDTA·2Na | 0.5 | 0.5 | 0.5 |
| Sterilized ion-exchanged water | to 100 | to 100 | to 100 |

A solution having the following composition was used as a gent No. 2. In addition, parts as a unit for expressing the amount to be incorporated means the parts by weight.

[Composition of an agent No. 2]

| Stearic acid | 1.0 part |
|---|---|
| Glyceryl monostearate | 1.5 part |
| Polyoxyethylene oleyl ether (20EO) | 1.0 part |
| Hydrogen peroxide (35%) | 15.5 parts |
| Sterilized ion-exchanged water | to 100 parts |

A tuft having a weight of 1 g and a length of 15 cm was dyed with a hair dye of Practical Product 5 or Comparative Product 9 - 10. Hair dyeing treatment was performed by blending the agents No. 1 and No. 2 in the same amount, applying the blend to the tuft, leaving it standing for 30 minutes, and rinsing with hot water followed by a 2% aqueous polyoxyethylene nonyl phenyl ether solution. After the hair dyeing treatment, the leveling of dyeing, gloss, moistness and combability of hair were evaluated by 10 panelers on the basis of the following criterion of five grades.

Evaluation criterion

Very good:    5,
Good:         4,
Average:      3,
Bad:          2,
Very bad:     1.

Furthermore, 30 hairs were removed from each of the tufts having been subjected to hair dyeing treatment, and the tensile strength of these hairs was measured to calculate the tensile strength per cross section. The results (average values) are shown in Table 19.

Table 19

| | Practical Product 5 | Comparative Product 9 | Comparative Product 10 |
|---|---|---|---|
| Hair after hair dyeing treatment | | | |
| Leveling | 4.5 | 4.0 | 1.8 |
| Gloss | 4.6 | 3.4 | 2.0 |
| Moistness | 4.5 | 3.2 | 2.0 |
| Combability | 4.6 | 3.4 | 1.6 |
| Tensile strength per cross section of hair (kgf/mm$^2$) | 29.8 | 27.7 | 26.2 |

As is apparent from the results in Table 19, the hair dye of Practical Product 5 having the yeast protein derived peptide composition in Example 3-1 got a higher score for all of the leveling, gloss, moistness and combability of hair which had been subjected to hair dyeing treatment over the hair dyes of Comparative Product 9 having the collagen derived peptide incorporated therein and Comparative Product 10 which was free of peptide. In addition, Practical Product 5 having the yeast protein derived peptide composition in Example 3-1 had an enhancement of about 14% in the tensile strength over Comparative Product 10, and thus it is indicated that the yeast protein derived peptide composition had an excellent sorption properties to hair.

Application Example 6

A body shampoo (Practical Product 6) shown in Table 20 was prepared by incorporating the yeast protein derived peptide composition (average molecular weight: 394) obtained in Example 4-1 therein. As the controls for comparison, a body shampoo (Comparative Product 11) having a collagen derived peptide (average molecular weight: 400) in place of the yeast protein derived peptide composition in Practical Product 6 incorporated therein and a body shampoo (Comparative Product 12) having neither of these peptide derivatives incorporated therein were prepared.

Table 20

|  | Practical Product 6 | Comparative Product 11 | Comparative Product 12 |
|---|---|---|---|
| Yeast protein derived peptide composition in Example 4-1 (average molecular weight: 394) (25%) | 8.0 | 0 | 0 |
| Collagen derived peptide (average molecular weight: 400) (PROMOIS W-32, manufactured by SEIWA KASEI, 30%) | 0 | 6.7 | 0 |
| Potassium salt of a condensate of a collagen derived peptide with a coconut oil fatty acid (PROMOIS ECP-C, manufactured by SEIWA KASEI, 35%) | 28.0 | 28.0 | 28.0 |
| Potassium soap of coconut oil (40%) | 7.5 | 7.5 | 7.5 |
| Coconut oil fatty acid diethanolamide | 3.0 | 3.0 | 3.0 |
| Lauric acid diethanolamide | 3.0 | 3.0 | 3.0 |
| Mixture of a p-hydroxybenzoic acid ester and phenoxyethanol (SEISEPT G, manufactured by SEIWA KASEI) | 0.3 | 0.3 | 0.3 |
| Sterilized ion-exchanged water | to 100 | to 100 | to 100 |

The smoothness, moistness, and stickyness of skin after washing with the body shampoo were evaluated by 10 panelers including 5 men and 5 women, who used the body shampoos over a period of 1 week (eight panelers used seven times and two panelers used four times) on the basis of the same criterion as in Application Example 1. The results are shown in Table 21.

Table 21

|  | Practical Product 6 | Comparative Product 11 | Comparative Product 12 |
|---|---|---|---|
| Skin after washing: |  |  |  |
| Smoothness | 2.0 | 1.0 | 0.0 |
| Moistness | 1.8 | 1.2 | 0.0 |
| Absence of stickiness | 0.7 | 0.7 | 1.6 |

As is apparent from the results in Table 21, Practical Product 6 having the yeast protein derived peptide composition in Example 4-1 afforded skin smoothness and moistness, and thus got a higher score over Comparative Product 11 having the collagten derived peptide incorporated therein as well as Comparative Product 12 which was free of peptide.

Although Comparative Product 12 having no peptide incorporated therein got the best score for evaluation of absence of stickiness, all of the panelers expressed the opinion that this product gave the feeling of drying and roughening to skin and no moistness.

Application Example 7

An after shaving lotion (Practical Product 7) shown in Table 22 was prepared with the yeast protein derived peptide composition (average molecular weight: 1,598) obtained in Example 1-2. As the controls for comparison, an after shaving lotion (Comparative Product 13) having a soybean protein derived peptide composition (average molecular weight: 1,500) in place of the yeast protein derived peptide composition in Practical Product 7 incorporated therein and an after shaving lotion (Comparative Product 14) having neither of these peptide derivatives incorporated therein were prepared.

Table 22

|  | Practical Product 7 | Comparative Product 13 | Comparative Product 14 |
|---|---|---|---|
| Yeast protein derived peptide composition in Example 1-2 (average molecular weight: 1,598) (25%) | 8.0 | 0 | 0 |
| Soybean protein derived peptide (average molecular weight: 1,500) (25%) | 0 | 8.0 | 0 |
| SEPIGEL 305 (trade name of thickener, manufactured by SEPPIC, France) | 1.5 | 1.5 | 1.5 |
| Talc | 5.0 | 5.0 | 5.0 |
| Ethanol (95%) | 10.0 | 10.0 | 10.0 |
| Mixture of a p-hydroxybenzoic acid ester and phenoxyethanol (SEISEPT G, manufactured by SEIWA KASEI) | 0.5 | 0.5 | 0.5 |
| Sterilized ion-exchanged water | to 100 | to 100 | to 100 |

The smoothness, moistness (moisturizing feeling), and stickyness of skin after the application of the after shaving lotion were evaluated by 5 male panelers, who used the products after shaving for 3 days per one product on the basis of the same criterion as in Application Example 1. The results are shown in Table 23.

Table 23

|  | Practical Product 7 | Comparative Product 13 | Comparative Product 14 |
|---|---|---|---|
| Skin after application: |  |  |  |
| Smoothness | 1.8 | 1.2 | 0.0 |
| Moistness (moisturizing feeling) | 1.7 | 1.3 | 0.0 |
| Absence of stickyness | 0.8 | 0.6 | 1.8 |

As is apparent from the results in Table 23 Practical Product 7 having the yeast protein derived peptide composition in Example 1-2 afforded skin smoothness and moistness, and thus got a higher score over Comparative Product 13 having the soybean protein derived peptide incorporated therein as well as Comparative Product 14 which was free of peptide.

Although Comparative Product 14 having no peptide incorporated therein got the best score for evaluation of absence of stickiness, all of the panelers expressed the opinion that this product afforded no stickyness but gave the feeling of drying and roughening to skin.

Application Example 8

A face washing agent (Practical Product 8) having the following composition was prepared by incorporating the yeast protein derived peptide composition (average molecular weight: 400) obtained in Example 4-2. As a control for comparison, a face washing agent (Comparative Product 15) having the same composition as in Practical Product 8 except no yeast protein derived peptide composition was prepared. In this connection, parts expressing the amount to be incorporated means parts by weight.

| | |
|---|---|
| Yeast protein derived peptide composition in Example 4-2 (average molecular weight: 400), (25%) | 4.0 parts |
| Potassium salt of a coconut oil fatty acid collagen hydrolysate (PROMOIS ECP-C, manufactured by SEIWA KASEI, 35%) | 25.0 parts |
| Stearic acid diethanolamide | 0.7 part |
| Coconut oil fatty acid diethanolamide | 5.0 parts |
| Polyoxyethylene oxide methylgucose diolate (120EO) | 0.5 part |
| Laurylaminopropionic acid (40%) | 3.0 parts |
| Coconut oil fatty acid amidopropylbetaine (30%) | 5.2 parts |
| EDTA·2Na | 0.1 part |
| Mixture of a p-hydroxybenzoic acid ester and phenoxyethanol (SEISEPT G, manufactured by SEIWA KASEI) | 0.5 part |
| Citric acid | to pH 6 |
| Sterilized ion-exchanged water | to 100 |

When washed with the face washing agent, Practical Product 8, having the yeast protein derived peptide composition in Example 4-2 incorporated therein, the agent gave skin no dry and roughening feeling. When compared with a face washing agent having no yeast protein derived peptide composition therein, Practical Product 8 afforded skin gloss and smoothness as well as good feelings during its use. These feelings is considered to be caused due to the sorption of the yeast protein derived peptide composition to skin and thus affording the skin the moisturizing effect.

The yeast protein derived peptide composition of the present invention is easy to control the molecular weight on preparing peptides. A yeast protein derived peptide composition having a stable molecular weight can be obtained with a good reproducibility, and the yeast protein derived peptide composition thus obtained is light in color, low in smell and excellent in stability during storage.

The yeast protein derived peptide composition of the present invention is thus incorporated in a variety of cosmetics such as a hair rinse, a hair treatment agent, a shampoo, a solution for permanent waving, a hair dyeing agent, a body shampoo, and a face washing agent. The yeast protein derived peptide composition as a component of these cosmetics is sorbed to hair and skin, affords hair and skin moistness and gloss, improves the smoothness of hair and skin, improves the combability of hair, and protect hair to prevent the damage of hair.

Thus, according to the present invention, yeast protein which has not hitherto been used as a peptide source for a raw material of cosmetics can be employed as a peptide source for a raw material of cosmetics, and the yeast protein derived peptide composition has excellent properties over the conventional protein hydrolysates.

**Claims**

1. A yeast protein derived peptide composition which is prepared by hydrolysing a yeast protein obtained by subjecting yeast cells to the heating treatment and the treatment with yeast cell wall lytic enzyme.

2. A yeast protein derived peptide composition according to claim 1, wherein heating of the yeast cells comprises heating an aqueous suspension of the yeast at a temperature from 50 - 100°C.

3. A yeast protein derived peptide composition according to claim 1 or 2, wherein heating of the yeast cells carried out before the treatment with the yeast cell wall lytic enzyme.

4. A yeast protein derived peptide composition according to any one of claims 1 - 3, wherein the treatment with the yeast cell wall lytic enzyme comprises adding the yeast cell wall lytic enzyme to an aqueous suspension of yeast at a concentration from 1 to 200 units per g of the yeast cell, and stirring the mixture at pH 5.0 - 8.0 at a temperature from 20 to 50°C.

5. A yeast protein derived peptide composition according to any one of claims 1 - 4, wherein the treatment with the yeast cell wall lytic enzyme is carried out in the presence of an alkali metal nitrite in the final concentration from 0.1 to 5 M.

6. A yeast protein derived peptide composition according to any one of claims 1 - 5, which has the average molecular weight from 200 to 5,000.

7. A process for preparing a yeast protein derived peptide composition, characterized in that yeast cells are subjected to the heating treatment and the treatment with a cell wall lytic enzyme, followed by separation and purification to give a yeast protein, which is then hydrolyzed.

8. A process according to Claim 7, wherein the heating of the yeast cell comprises heating an aqueous suspension of the yeast to a temperature from 50 - 100°C.

9. A process according to Claim 7 or 8, wherein heating of the yeast cells carried out before the treatment with the yeast cell wall lytic enzyme.

10. A process according to any one of Claims 7 - 9, wherein the treatment with the yeast cell wall lytic enzyme comprises adding the yeast cell wall lytic enzyme to an aqueous suspension of yeast at a concentration from 1 to 200 units per g of the yeast cell, and stirring the mixture at pH 5.0 - 8.0 at a temperature from 20 to 50°C.

11. A process according to any one of Claims 7 - 10, wherein the treatment with the yeast cell wall lytic enzyme is carried out in the presence of an alkali metal nitrite in the final concentration from 0.1 to 5 M.

12. A component for a cosmetic comprising the yeast protein derived peptide composition according to any one of claims 1 - 6.

13. A humectant for a cosmetic comprising the yeast protein derived peptide composition according to any one of claims 1 - 6.

14. A hair treatment agent, wherein the yeast protein derived peptide composition according to any one of claims 1 - 6 has been incorporated.

15. A shampoo, wherein the yeast protein derived peptide composition according to any one of claims 1 - 6 has been incorporated.

16. An agent No. 1 for permanent waving, wherein the yeast protein derived peptide composition according to any one of claims 1 - 6 has been incorporated.

17. A hair dyeing agent, wherein the yeast protein derived peptide composition according to any one of claims 1 - 6 has been incorporated.

18. A skin washing agent, wherein the yeast protein derived peptide composition according to any one of claims 1 - 6 has been incorporated.

19. Use of a yeast protein derived peptide composition according to any one of claims 1 - 6 for the preparation of a cosmetic.

FIG. 1

FIG. 2